# EUROPEAN PATENT APPLICATION

(11) **EP 4 026 584 A1**
(43) Date of publication of application: **13.07.2022**
(21) Application number: 21150911.2
(22) Date of filing: 11.01.2021
(51) Int. Cl.: A61N 1/04, A61B 5/055, A61N 7/00, A61B 5/00

(54) **NEUROMODULATION OF A PATIENT'S BRAIN WITH A HEAD COIL DEVICE**

(71) Applicant: Koninklijke Philips N.V., 5656 AG Eindhoven (NL)
(72) Inventor: MATTERS, Marco, 5656 AE Eindhoven (NL); LAMERICHS, Rudolf Mathias Johannes Nicolaas, 5656 AE Eindhoven (NL); LEUFKENS, Timmy Robertus Maria, 5656 AE Eindhoven (NL); VAN EE, Raymond, 5656 AE Eindhoven (NL); HUIJBERS, Willem, 5656 AE Eindhoven (NL)
(74) Representative: Philips Intellectual Property & Standards

(57) **Abstract**

The invention relates to neuromodulation of a patient's brain with a head coil device- The head coil device according to the invention is adapted for a functional magnetic resonance imaging, fMRI, system (2), wherein the head coil device (5) is configured to be placed on a patient's head (6) and comprises a plurality of receive coils (8) for receiving fMRI signals from the patient's head (6), and electrodes (9) and/or ultrasonic transducers (10) for neuromodulation of at least a part of the brain of the patient (7). In this way, a possibility for a well controllable and easily reproducible neuromodulation of the human brain is provided.

## Description

### FIELD OF THE INVENTION

The invention relates to the field of functional magnetic resonance imaging (fMRI), and in particular to the neuromodulation of at least a part of the brain of a patient.

### BACKGROUND OF THE INVENTION

Non-invasive neuromodulation of brain areas with external electrodes by transcranial direct current stimulation (tDCS) or transcranial alternating current stimulation (tACS), or transcranial functional ultrasound stimulation (tFUS) by ultrasound with ultrasound transducers is commonly used in scientific studies and in treatment of a range of neurological and psychiatric disorders. These methods are similar to transcranial magnetic stimulation, but use electrical current or ultrasound to evoke neuronal activity. Further, functional ultrasound imaging (fUS) is known for indirectly measuring cerebral blood volume for a map of brain activation due to external stimuli.

Functional magnetic resonance imaging measures brain activity by detecting changes associated with blood flow. This technique relies on the fact that cerebral blood flow and neuronal activation are coupled. When an area of the brain is in use, blood flow to that region also increases. In a typical form of fMRI the blood-oxygen-level dependent (BOLD) contrast is used. With a respective brain scan it is possible to map neural activity in the brain of a humans by imaging the change in blood flow (hemodynamic response) related to energy use by brain cells. In this respect, further types of fMRI are arterial spin labeling (ASL) fMRI and vascular space occupancy (VASO) fMRI.

Conventional methods for non-invasive neuromodulation of the human brain suffer from difficult controllability and poor reproducibility.

From US 2015/0099963 A1 a method for transcranial magnetic stimulation (TMS) of a stimulation area of medical interest, combined with functional magnetic resonance imaging (fMRI) for visualization of the response of, for example neurons, is known. An ultra-thin magnetic resonance coil, MR coil, positioned in the immediate vicinity over an area where the response of, for example neurons, is to be detected, and preferably sandwiched between the TMS coil and the area, provides an excellent signal-to-noise ratio. The TMS can be performed directly through the MR coil. A great deal of flexibility in the number of the TMS and MR coils in use and their spatial arrangement is provided. A corresponding system for the TMS/fMRI studies is also provided.

Further, US 2012/123245 A1 describes a modular multi-transmit head coil for use in deep brain stimulation procedures which includes a mounting frame arranged for attachment to the stereotactic head frame of the patient. An integrated MR stereotactic locating phantom is included as part of the coil structure which has attached MR/CT compatible fiducials on it to correlate pre-op, intra-op and post op images. The frame carries a series of modular removable coil elements in a transceive array that are connected to a set of RF power amplifiers that directly drive the coil elements. A control system including suitable software is arranged to control the signals to the coil elements so as to generate a required RF field with all the coil elements in place and with one or more the coil elements removed to allow access to the patent for the installation of the DBS leads.

### SUMMARY OF THE INVENTION

It is an object of the invention to provide a possibility for a well controllable and easily reproducible neuromodulation of the human brain.

According to the invention, this object is addressed by the subject matter of the independent claims. Preferred embodiments of the invention are defined in the dependent claims.

Therefore, according to the invention, a head coil device for a functional magnetic resonance imaging, fMRI, system, is provided, wherein the head coil is configured to be placed on a patient's head and comprises a plurality of receive coils for receiving fMRI signals from the patient's head, and electrodes and/or ultrasonic transducers for neuromodulation of at least a part of the brain of the patient.

With this head coil device according to the invention, non-invasive neuromodulation of brain areas with the electrodes of the head coil device by transcranial direct current stimulation or transcranial alternating current stimulation and/or by ultrasound signals with ultrasound transducers of the head coil device becomes possible with simultaneous functional ultrasound imaging by which cerebral blood volume may indirectly be measured for generating a map of brain activation due to the external stimuli from the electrodes and/or ultrasound transducers. While conventional neuromodulation is commonly done without feedback from areas of the brain that are in fact modulated, neuromodulation with functional MRI feedback can optimize treatment that is dependent on targeting the correct brain area. The head coil device according to the invention with integrated electrodes and or ultrasound transducers makes such neuromodulation easily feasible. Here, functional MRI (fMRI) means all types of fMRI, i.e. any MRI sequences that may be used for measuring response, especailly including BOLD fMRI, VASO fMRI and ASL fMRI.

In general, the receive coils and the electrodes and/or the ultrasonic transducers may be fixed to the head coil device in different ways. According to a preferred embodiment of the invention, the head coil device comprises a flexible carrier which carries the receive coils and the electrodes and/or the ultrasonic transducers. In this way, it can be made possible that the head coil device is worn on the patient's head in such a way that the electrodes and/or the ultrasonic transducers make direct contact with the head and that this direct contact of the electrodes and/or the ultrasonic transducers with the head is maintained even when the patient moves his head. With respect to the flexible carrier, it is further preferred that the flexible carrier is made of an electrical insulating material. In this way, it can be avoided that the receive coils and the electrodes and/or the ultrasonic transducers affect each other under operating conditions. In this respect, according to a preferred embodiment of the invention, the receive coils and the electrodes and/or the ultrasonic transducers are galvanically insulated from each other.

According to a preferred embodiment of the invention, the head coil device comprises at least one motion sensor for tracking movements of the patient's head. In this way, it becomes possible to measure the movement of the patient's head in space, e.g. relative to a fMRI device which is used for the fMRI method for analyzing the effects of the external stimuli to the patient. Such measurements of the patient's movements relative to the fMRI device may be used for compensating effects which are due to these movements.

Further, the invention relates to a system for neuromodulation of a patient's brain, wherein the system comprises a functional magnetic resonance imaging system for acquiring fMRI images of the patient's brain with a head coil as described before, and an analysis unit for analyzing the effected neuromodulation of the patient's brain on the basis of the acquired fMRI images relative to a predefined intended neuromodulation of the patient's brain.

According to a preferred embodiment of the invention, this system for neuromodulation of a patient's brain further comprises a control unit for controlling the electrodes and/or ultrasonic transducers in dependence of the deviation of the effected neuromodulation of the patient's brain from the predefined intended neuromodulation of the patient's brain. In this way, it becomes possible to control the neuromodulation of the patient's brain in such a way that deviations from a predefined intended neuromodulation are further reduced. In this respect, it is further preferred that the analysis unit is adapted for analyzing the effected neuromodulation of the patient's brain in real time.

Furthermore, the invention also relates to a method for neuromodulation of a patient's brain, wherein the method comprises the following method steps: arranging electrodes and/or ultrasonic transducers on the patient's head, neuromodulating at least a part of a patient's brain with an initial set of the electrodes and/or ultrasonic transducers, acquiring fMRI images from the patient's brain, analyzing the effected neuromodulation of the patient's brain on the basis of the acquired fMRI images relative to a predefined intended neuromodulation of the patient's brain, and controlling the electrodes and/or ultrasonic transducers in dependence of the deviation of the effected neuromodulation of the patient's brain from the predefined intended neuromodulation of the patient's brain. As set out before, it is preferred that the step of analyzing the effected neuromodulation of the patient's brain on the basis of the acquired fMRI images relative to a predefined intended neuromodulation of the patient's brain is executed in real time.

According to a preferred embodiment of the invention, the step of controlling the electrodes and/or ultrasonic transducers in dependence of the deviation of the effected neuromodulation of the patient's brain from the predefined intended neuromodulation of the patient's brain is executed by activating a new set of the electrodes and/or ultrasonic transducers which is different from the initial set of electrodes and/or ultrasonic transducers, and or activating the initial set of electrodes and/or ultrasonic transducers in a different way, and/or changing the position of the electrodes and/or ultrasonic transducers on the patient's head. In this respect, activating a new set of the electrodes and/or ultrasonic transducers which is different from the initial set of electrodes and/or ultrasonic transducers means that the new set of the electrodes and/or ultrasonic transducers differs from the initial set of electrodes and/or ultrasonic transducers at least in one electrode and/or ultrasonic transducer. Therefore, the new set of the electrodes and/or ultrasonic transducers comprises at least one more electrode and/or ultrasonic transducer, or comprises at least one less electrode and/or ultrasonic transducer, or comprises at least one other electrode and/or ultrasonic transducer. Activating the initial set of electrodes in a different way means that at least one of the electrodes is controlled with a different current, i.e. a current which may differ in amplitude and/or frequency. Activation of the initial set of ultrasonic transducers in a different way means controlling at least one of the ultrasonic transducers such that the ultrasonic signal output by the respective ultrasonic transducer differs from the previous signal in amplitude and/or frequency. Changing the position of the electrodes and/or ultrasonic transducers on the patient's head means that the position of at least one electrode and/or at least one ultrasonic transducer is changed to a new position.

According to a preferred embodiment of the invention, the step of arranging the electrodes and/or ultrasonic transducers on the patient's head comprises a step of arranging a head coil device on the patient's head which comprises a plurality of receive coils for receiving fMRI signals from the patient's head, and the electrodes and/or ultrasonic transducers for neuromodulation of at least a part of the brain of the patient. Using such a head coil device has multiple advantages as set out further above.

Further, it is preferred that the method comprises the additional step of tracking movements of the patient's head with at least one motion sensor which is comprised by the head coil device which is arranged on the patient's head. As also set out further above, in this way it becomes possible to measure the movement of the patient's head in space, e.g. relative to a fMRI device which is used for the fMRI method for analyzing the effects of the external stimuli to the patient. Then, such measurements of the patient's movements relative to the fMRI device can be used for compensating effects which are due to these movements. In this respect, according to a preferred embodiment of the invention, the method comprises the additional step of correcting the acquired fMRI images on the basis of the tracked movements of the patient's head.

The invention also relates to a non-transitory computer-readable medium, comprising instructions stored thereon, that when executed on a processor induce a system as described above to perform a method as described above.

### BRIEF DESCRIPTION OF THE DRAWINGS

These and other aspects of the invention will be apparent from and elucidated with reference to the embodiments described hereinafter. Such an embodiment does not necessarily represent the full scope of the invention, however, and reference is made therefore to the claims and herein for interpreting the scope of the invention.

In the drawings:
Fig. 1 schematically depicts a system for neuromodulation of a patient's brain according to a preferred embodiment of the invention,
Fig. 2 schematically depicts a head coil with electrodes according to a preferred embodiment of the invention,
Fig. 3 schematically depicts a head coil with ultrasonic transducers according to a preferred embodiment of the invention, and
Fig. 4 schematically depicts a method for neuromodulation of a patient's brain according to a preferred embodiment of the invention.

### DETAILED DESCRIPTION OF EMBODIMENTS

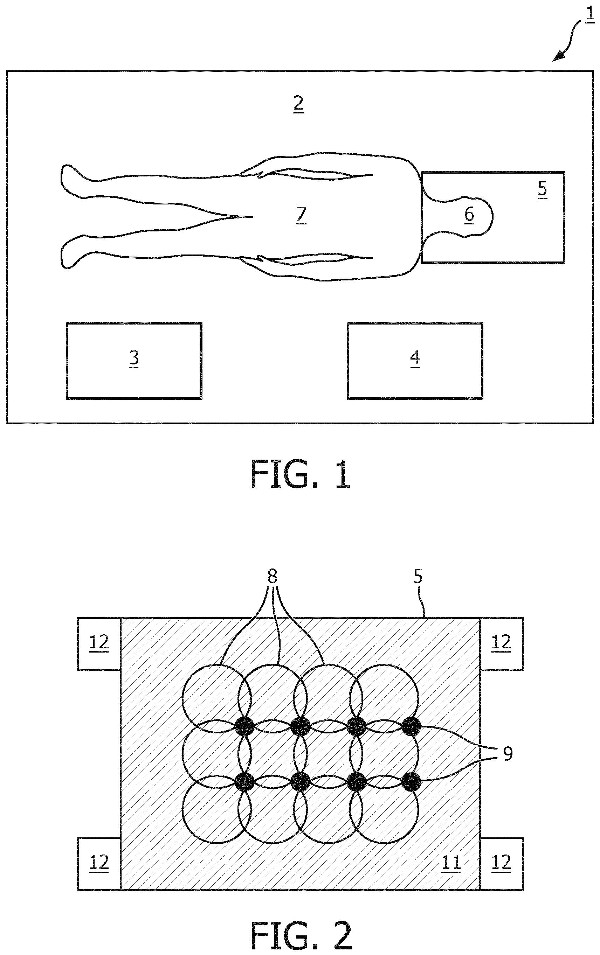

Fig. 1 schematically depicts a system 1 for neuromodulation of a patient's brain according to a preferred embodiment of the invention. This system 1 comprises a functional magnetic resonance imaging system (fMRI) 2 for acquiring fMRI images of the patient's brain with a head coil device 5. Further, the system comprises an analysis unit 3 and a control unit 4.

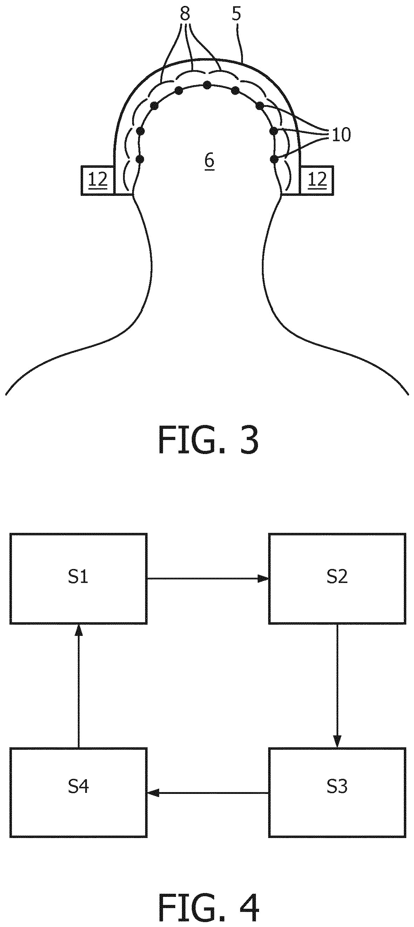

As schematically depicted in Figs. 1 and 3, the head coil device 5 is configured to be placed on a head 6 of a patient 7. From Fig. 2 it can be seen that the head coil device 5 according to this preferred embodiment of the invention comprises a plurality of receive coils 8 for receiving fMRI signals from the patient's head 6, and electrodes 9 for neuromodulation of at least a part of the brain of the patient 7. The head coil device 5 further comprises a flexible carrier 11, in Fig. 2 depicted by the hatched areas, which carries the receive coils 8 and the electrodes 9. The carrier 11 is made of an electrical insulating material, and the receive coils 8 and the electrodes 9 are arranged on the carrier 11 such that they are galvanically insulated from each other. Further, as also depicted in Fig. 2, the head coil device 5 comprises motion sensors 12 for tracking movements of the patient's head 6. When the head coil device 5 is worn on the patient's head 6, the electrodes 9 come in direct contact with the patient's head 6. In this way, these electrodes 9 may be used for non-invasive neuromodulation of brain areas by transcranial direct current stimulation (tDCS) or transcranial alternating current stimulation (tACS).

Fig. 3 shows an alternative preferred embodiment of the invention. The head coil device 5' according to this preferred embodiment of the invention is designed in the same way as the head coil device which is schematically depicted in Fig. 2 except for the fact that instead of electrodes 9 ultrasonic tranducers 10 are provided for neuromodulation of at least a part of the brain of the patient 7. Both head coil devices 5, 5' can generally be used in the same way with the obvious difference that the electrodes 9 are used for applying a current to the brain of the patient 7 while the ultrasonic trandsducers 10 are used to apply ultrasonic signals to the brain of the patient 7. Except for this difference, the general method of using these head coil devices 5, 5' as described herein may be the same.

Therefore, the analysis unit 3 is adapted for analyzing the effected neuromodulation of the patient's brain on the basis of the acquired fMRI images relative to a predefined intended neuromodulation of the patient's brain in real time while the functional magnetic resonance imaging system 2 is used for fMRI measurment of the brain activity by detecting changes associated with blood flow. The control unit 4 is adapted for controlling the electrodes 9 or the ultrasonic transducers 10, respectively, in dependence of the deviation of the effected neuromodulation of the patient's brain from a predefined intended neuromodulation of the patient's brain.

The method for neuromodulation of a patient's brain according to a preferred embodiment of the invention is as follows:
First, a head coil device 5, 5' as described above is arranged on the head 6 of the patient 7. As set out above, the head coil device 5, 5' comprises a plurality of receive coils 8 for receiving fMRI signals from the patient's head 6, and the electrodes 9 or ultrasonic transducers 10 for neuromodulation of at least a part of the brain of the patient 7.

When the head coil device 5, 5' is arranged on the patient's head 6, at least a part of the patient's brain is neuromodulated with an initial set of the electrodes 9 or an initial set of ultrasonic transducers 10. This initial set of electrodes 9 or this initial set of ultrasonic transducers 10 is selected according to an intended neuromodulation of the patient's brain which is used as a predefined value in the following. For example, this initial selection may be done randomly or on the basis of a lookup table which provides different sets of electrodes 9 or ultrasonic transducers 10 for certain intended neuromodulations. During this neuromodulation fMRI images are acquired from the patient's brain with the aid of the functional magnetic resonance imaging system 2.

Then, the effected neuromodulation of the patient's brain is analyzed on the basis of the acquired fMRI images relative to a predefined intended neuromodulation of the patient's brain in real time which offers the possibility for controlling the electrodes 9 or ultrasonic transducers 10 in dependence of the deviation of the effected neuromodulation of the patient's brain from the predefined intended neuromodulation of the patient's brain.

This step of controlling the electrodes 9 or ultrasonic transducers 10 in dependence of the deviation of the effected neuromodulation of the patient's brain from the predefined intended neuromodulation of the patient's brain may be executed in different ways. For example, it is possible to activate a new set of the electrodes 9 or ultrasonic transducers 10 which is different from the initial set of electrodes 9 or ultrasonic transducers 10. Alternatively, or in addition, it is possible to activate the initial set of electrodes 9 or ultrasonic transducers 10 in different way, i.e. for outputting a current or ultrasonic signal, respectively, which differs in amplitude and/or frequency from the previous one. Further, alternatively or in addition, it is possible to change the position of the electrodes 9 or ultrasonic transducers lOon the patient's head 6 which means that at least one electrode 9 or ultrasonic transducer 10 is arranged at a different position.

Furthermore, with the aid of the motion sensors 12 of the head coil devices 5, 5', it is possible to track movements of the patient's head 6. This provides for the possibility of correcting the acquired fMRI images on the basis of the tracked movements of the patient's head 6.

Summarized and with reference to Fig. 4, according to a preferred embodiment of the invention, the following method is applied: In step S1 neuromodulation and fMRI is performed simultaneously. Then, in step S2 real-time analysis of the acquired fMRI images is done in order to determine the achieved neuromodulation. In this way, in step S3, a feedback may be achieved by comparing the achieved neuromodulation with the intended neuromodulation, which makes it possible to provide the combined neuromodulation and fMRI system in step S4 with amended control signals for driving the electrodes 9 or ultrasonic transducers 10 in order to reduce the deviations of the achieved neuromodulation with the intended neuromodulation.

A special application of the present invention may be as follows: It is known that DCS effects depend on the baseline status of the brain at the time of its application. This dependency could possibly influence interindividual variability in DCS effects previously reported, and the fact that differences in experimental protocols such as stimulation intensity or use of different versions of the same or different behavioral tasks, result in different outcomes. In this respect, with simultaneous and real-time functional MRI with a head coil device according to the invention, the present invention allows controlling for these variations which makes it possible to provide a more standardized but still personalized way of neuromodulation treatment.

While the invention has been illustrated and described in detail in the drawings and foregoing description, such illustration and description are to be considered illustrative or exemplary and not restrictive; the invention is not limited to the disclosed embodiments. Especially, though only examples of head coil devices 5, 5' have been described which either comprise electrodes 9 or ultrasonic transducers 10, the invention also offer the possibility of providing a head coil device which comprises electrodes 9 and ultrasonic transducers. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope. Further, for the sake of clearness, not all elements in the drawings may have been supplied with reference signs.

### REFERENCE SYMBOL LIST

| | |
|---|---|
| system for neuromodulation of a patient's brain | 1 |
| functional magnetic resonance imaging system | 2 |
| analysis unit | 3 |
| control unit | 4 |
| head coil device | 5, 5' |
| head of the patient | 6 |
| patient | 7 |
| receive coils | 8 |
| electrodes | 9 |
| ultrasonic transducers | 10 |
| carrier | 11 |
| motion sensors | 12 |

## Claims

1. A head coil device (5) for a functional magnetic resonance imaging, fMRI, system (2), wherein the head coil device (5) is configured to be placed on a patient's head (6) and comprises
a plurality of receive coils (8) for receiving fMRI signals from the patient's head (6), and
electrodes (9) and/or ultrasonic transducers (10) for neuromodulation of at least a part of the brain of the patient (7).

2. The head coil device (5) according to claim 1, wherein the head coil device (5) comprises a flexible carrier (11) which carries the receive coils (8) and the electrodes (9) and/or the ultrasonic transducers (10).

3. The head coil device according to claim 2, wherein the flexible carrier (11) is made of an electrical insulating material.

4. The head coil device (5) according to any of claims 1 to 3, wherein the receive coils (8) and the electrodes (9) and/or the ultrasonic transducers (10) are galvanically insulated from each other.

5. The head coil device (5) according to any of claims 1 to 4, wherein the head coil device (5) comprises at least one motion sensor (12) for tracking movements of the patient's head (6).

6. A system for neuromodulation of a patient's brain, wherein the system comprises
a functional magnetic resonance imaging system (2), fMRI, for acquiring fMRI images of the patient's brain with a head coil device (5) according to any of claims 1 to 5, and
an analysis unit (3) for analyzing the effected neuromodulation of the patient's brain on the basis of the acquired fMRI images relative to a predefined intended neuromodulation of the patient's brain.

7. The system according to claim 6, further comprising
a control unit (4) for controlling the electrodes (9) and/or ultrasonic transducers (10) in dependence of the deviation of the effected neuromodulation of the patient's brain from the predefined intended neuromodulation of the patient's brain.

8. The system according to claim 6 or 7, wherein the analysis unit (3) is adapted for analyzing the effected neuromodulation of the patient's brain in real time.

9. A method for neuromodulation of a patient's brain, wherein the method comprises the following method steps:
arranging electrodes (9) and/or ultrasonic transducers (10) on the patient's head (6),
neuromodulating at least a part of a patient's brain with an initial set of the electrodes (9) and/or an initial set of ultrasonic transducers (10),
acquiring fMRI images from the patient's brain,
analyzing the effected neuromodulation of the patient's brain on the basis of the acquired fMRI images relative to a predefined intended neuromodulation of the patient's brain, and
controlling the electrodes (9) and/or ultrasonic transducers (10) in dependence of the deviation of the effected neuromodulation of the patient's brain from the predefined intended neuromodulation of the patient's brain.

10. The method according to claim 9, wherein the step of analyzing the effected neuromodulation of the patient's brain on the basis of the acquired fMRI images relative to a predefined intended neuromodulation of the patient's brain is executed in real time.

11. The method according to claim 9 or 10, wherein the step of controlling the electrodes (9) and/or the ultrasonic transducers (10) in dependence of the deviation of the effected neuromodulation of the patient's brain from the predefined intended neuromodulation of the patient's brain is executed by
activating a new set of the electrodes (9) and/or a new set of ultrasonic transducers (10) which is different from the initial set of electrodes (9) and/or the initial set of ultrasonic transducers (10), and or
activating the initial set of electrodes (9) and/or the initial set of ultrasonic transducers (10) in a different way, and/or
changing the position of the electrodes (9) and/or the ultrasonic transducers (10) on the patient's head.

12. The method according to any of claims 9 to 11, wherein the step of arranging the electrodes (9) and/or the ultrasonic transducers (10) on the patient's head comprises a step of arranging a head coil device (5) on the patient's head (6) which comprises a plurality of receive coils (8) for receiving fMRI signals from the patient's head (6), and the electrodes (9) and/or the ultrasonic transducers (10) for neuromodulation of at least a part of the brain of the patient (7).

13. The method according to claim 12, wherein the method comprises the additional step of
tracking movements of the patient's head (6) with at least one motion sensor (12) which is comprised by the head coil device (5) which is arranged on the patient's head (6).

14. The method according to claim 13, wherein the method comprises the additional step of
correcting the acquired fMRI images on the basis of the tracked movements of the patient's head (6).

15. A non-transitory computer-readable medium, comprising instructions stored thereon, that when executed on a processor induce a system according to claim 6 to 8 to perform a method according to any of claims 9 to 14.
